# EUROPEAN PATENT APPLICATION

(11) **EP 3 067 077 A1**
(43) Date of publication of application: **14.09.2016**
(21) Application number: 15290063.5
(22) Date of filing: 09.03.2015
(51) Int. Cl.: A61M 5/14, G06F 19/00

(54) **SYSTEM COMPRISING A MULTIPLICITY OF MEDICAL DEVICES AND A CENTRAL DISPLAY CONTROL DEVICE**

(71) Applicant: Fresenius Vial SAS, 38590 Brézins (FR)
(72) Inventor: Grube, Frank, 38690 Biol (FR); Cuadrado, Virginie, 80331 München (DE)
(74) Representative: Kusche, Robert

(57) **Abstract**

A system (1) comprises at least one organization device (2, 3) for arranging a multiplicity of medical devices (20, 30) and for providing a communication link for the multiplicity of medical devices (20, 30). A multiplicity of medical devices (20, 30) is arranged on the at least one organization device (2, 3), each medical device (20, 30) comprising a display (200) for displaying operational information of the medical device (20, 30). A control device (7) is communicatively connected with the at least one organization device (2, 3) and the multiplicity of medical devices (20, 30) arranged on the at least one organization device (2, 3), wherein the control device (7) comprises an input device (70) for allowing to select by a user at least one of the multiplicity of medical devices (20, 30) for inputting a control command for controlling the at least one selected medical device (20, 30). Herein, the control device (7) is constituted to generate and transmit, upon selection of at least one of the multiplicity of medical devices (20, 30) via the input device (70) of the control device (7), at least one control signal (S) for causing a visual signal (V) to appear at the selected at least one medical device (20A, 20B) and/or at the other, non-selected medical devices of the multiplicity of medical devices (20, 30) to visually differentiate the selected at least one medical device (20A, 20B) from the other, non-selected medical devices.

## Description

The invention relates to a system according to the preamble of claim 1 and to a method for operating a system.

Such system comprises at least one organization device for arranging a multiplicity of medical devices and for providing a communication link for the multiplicity of medical devices. Within the system a multiplicity of medical devices is arranged on the at least one organization device, wherein each medical device comprises a display for displaying operational information of the medical device. A control device is connected with the at least one organization device and the multiplicity of medical devices for communicating with the at least one organization device and the multiplicity of medical devices arranged on the at least one organization device. The control device comprises an input device which allows a user to select at least one of the multiplicity of medical devices for inputting a control command for controlling the selected at least one medical device.

An organization device of this kind may, for example, be constituted by a rack on which medical devices, for example infusion pumps, can be arranged to form a vertical stack. A rack of this kind may be constituted to receive a large number of medical devices, for example eight or twelve medical devices, wherein multiple racks may be used in combination. By means of such racks medical devices such as infusion pumps can be arranged for example at the bedside of a patient in a hospital environment in an organized fashion.

The medical devices arranged on such organization devices typically can be controlled via a central control device, which may be part of the organization device, for example the rack, or may be provided as a separate unit in addition to the organization device and connected to the organization device via a data link or a hospital data network. The control device is connected with the organization devices and with the medical devices arranged on the organization devices such that data can be exchanged between the control device and the medical devices arranged on the organization devices.

In a state-of-the-art system the control device may comprise a touch-sensitive display via which a user can enter control commands relating to a single medical device or a group of medical devices arranged on the organization devices. The display of the control device may include a graphical representation of the medical devices arranged on the organization devices, allowing a user to address and control medical devices or groups of medical devices via the control device.

Because the control device is spatially separated from the medical devices arranged on the organization devices, it may not be easily possible for a user to associate a medical device selected via the input device of the control device with the actual physical device. This becomes even more complicated if a large number of medical devices are used on multiple, spatially separated organization devices forming, for example, multiple stacks of medical devices. There hence is a desire to facilitate the use of a complex system including a multiplicity of medical devices for a user such that the user can easily and intuitively associate input commands entered via the central control device with actual physical medical devices.

WO 2009/079623 A1 teaches a system in which a user can centrally control the brightness level of medical devices arranged for example in a clinical care area.

It is an object of the instant invention to provide a system and a method allowing for an easy and intuitive control of a multiplicity of medical devices via a central control device.

This object is achieved by means of a system comprising the features of claim 1.

Herein, the control device is constituted to generate and transmit, upon selection of at least one of the multiplicity of medical devices via the input device of the control device, at least one control signal for causing a visual signal to appear at the selected at least one medical device and/or at the other, non-selected medical devices of the multiplicity of medical devices to visually differentiate the selected at least one medical device from the other, non-selected medical devices.

The general procedure is as follows. A user selects via the input device of the control device, for example via a touch-sensitive display of the control device, an individual medical device or a group of medical devices arranged on one or multiple organization devices, in particular racks forming vertical stacks of medical devices. Upon selection of an individual medical device or a group of medical devices the user may enter a control command for controlling the individual medical device or the group of medical devices. The control command may for example relate to an operational setting of the individual medical device or the group of medical devices to influence for example, infusion parameters such as a flow rate, a bolus, a duration of administration or the like.

Once the user has selected an individual medical device or a group of medical devices, the control device - automatically without further user interaction - generates a control signal and transmits it to the organization devices and the medical devices arranged thereon. The control signal causes a visual signal to appear for example at the individual medical device or the group of medical devices which have been selected, such that the user immediately upon selection of the medical devices receives a visual feedback which medical devices have been selected.

The visual signal herein appears at the individual medical device or the group of medical devices which have been selected, or it appears at the other of the multiplicity of medical devices which have not been selected. The visual signal causes a visual differentiation between the selected and the non-selected medical devices such that the user is informed which physical medical devices are associated with the selection entered via the input device of the control device and which are not.

The visual signal which is caused to appear at the selected medical devices and/or the non-selected medical devices may have different shapes. If the visual signal appears at the selected medical devices, the shape of the visual signal beneficially is the same at all selected medical devices.

In one embodiment, the visual signal may be caused by an adjustment of the brightness value of the display of the selected medical devices. Herein, in a default state the control device beneficially controls the displays of all medical devices arranged on the organization devices and communicatively connected and associated with the control device to be homogeneous. In particular, in a default state the displays of the medical devices are controlled to have a brightness equal to a default brightness value.

The default brightness value herein may be user selectable via the control device.

The brightness value may in particular relate to a backlight brightness of the display, i.e. to background portions of the display.

Upon selection of an individual medical device or a group of medical devices via the input device of the control device, the control device may then generate a control signal which causes the brightness value of the selected medical devices to be increased above the default brightness value. Upon selection of an individual or a group of medical devices, the displays of the selected medical devices hence shine up such that a user is informed which actual physical medical devices are associated with the selection the user has entered via the input device of the control device.

This visual effect may further be increased by decreasing the brightness value of the displays of those medical devices which have not been selected. The displays of the non-selected medical devices hence may be dimmed in relation to the selected medical devices, such that the visual effect at the selected medical devices is further emphasized.

It is possible that, in one embodiment, a visual signal appears only at the non-selected medical devices. For example, by dimming the brightness of the displays of the non-selected medical devices a visual differentiation between the selected medical devices and the non-selected medical devices may be caused which is clearly visible for a user.

In principle, other visual signals may be displayed at the selected medical devices. For example, in addition or instead of adjusting the brightness of the display, the background color of the displays of the selected medical devices may be caused to change (for example from grey to red or the like), or a rectangular box or another visual mark may appear on the display, hence indicating the selection of the actual physical medical device.

A visual signal may also appear at another location remote from the display on a selected medical device. For example, a button or knob, in particular a rotary knob, may be caused to shine up or change color.

The visual signal to appear at the selected medical devices may be constant over at least a predetermined amount of time, e.g. the brightness of the displays of the selected medical devices is constantly high over the pre-determined amount of time. Instead, however, it is also possible to use a time-varying visual signal. For example, a display may be blinking by periodically changing the brightness or by periodically reversing the contrast of the display. Or a rectangular box surrounding the display may blink in a periodic fashion.

Beneficially, the visual signal disappears after lapse of a pre-determined time period or after a cancellation command is input by a user at the control device. Hence, after a predetermined time or after a cancellation command, the selected medical devices revert to a default state, e.g. the brightness of the displays of the selected medical devices is reset to the default brightness value, and/or a visual mark such as a rectangular box on the displays or the lighting of a button or knob vanishes.

The object is also achieved by a method for operating a system. The system herein comprises at least one organization device for mechanically holding a multiplicity of medical devices and for providing a communication link for the multiplicity of medical devices. The system further comprises a multiplicity of medical devices arranged on the at least one organization device, wherein each medical device comprises a display for displaying operational information of the medical device. A control device is communicatively connected with the at least one organization device and the multiplicity of medical devices arranged on the at least one organization device. Herein, within the method a user selects, via an input device of the control device, at least one of the multiplicity of medical devices for inputting a control command for controlling the at least one selected medical device. Upon selection of at least one of the multiplicity of medical devices via the input device of the control device, the control device generates and transmits at least one control signal, wherein the control signal causes a visual signal to appear at the selected at least one medical device and/or at the other, non-selected medical devices of the multiplicity of medical devices to visually differentiate the selected at least one medical device from the other, non-selected medical devices.

The advantages and advantageous embodiments described above with regard to the system equally apply also to the method such that it shall be referred to the above.

The idea underlying the invention shall subsequently be described in more detail with regard to the embodiments shown in the figures. Herein
- Fig. 1: shows a schematic view of a multiplicity of medical devices arranged at a bed side of a patient in a hospital environment;
- Fig. 2: shows a view of a system comprising multiple organization devices in the shape of racks, each rack holding a multiplicity of medical devices, and a control device for entering control commands relating to the medical devices; and
- Fig. 3: shows an example of a view on a display of the control device.

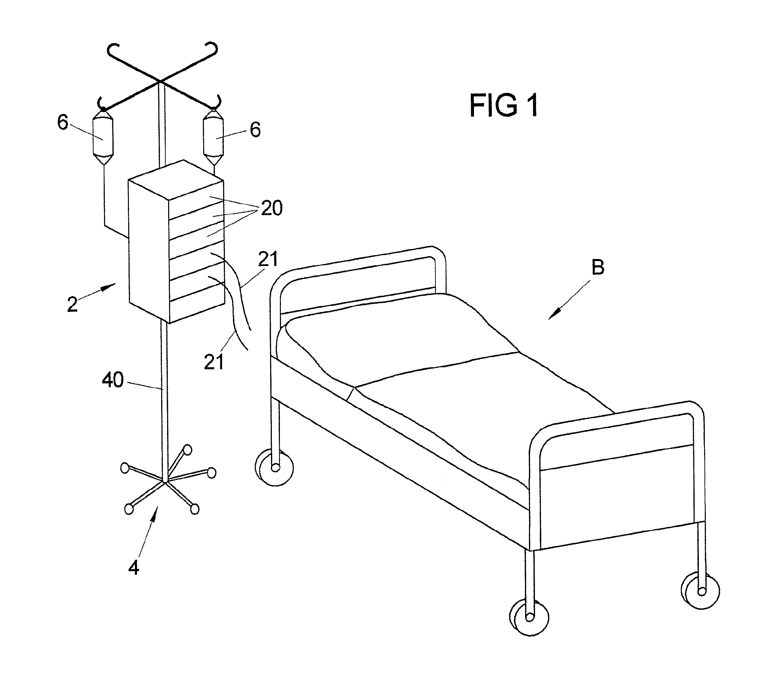

Fig. 1 shows in a schematic drawing a scenario as it typically can be found in a hospital environment, for example an intensive care unit of a hospital. Next to the bed B of a patient a number of medical devices 20 constituted for example as infusion pumps, such as syringe pumps or volumetric pumps, are located and connected to a patient via infusion lines 21. The medical devices 20 serve to administer a fluid such as a medication or nutrients for example contained in containers 6 via infusion lines 21 to the patient, the infusion lines 21 (especially in the environment of an intensive care unit of a hospital) possibly being vital to the patient such that they under all conditions must remain connected to the patient to ensure the required administration of medication, nutrients or the like.

Typically, the medical devices 20 constituted as infusion pumps are organized in a rack 2 to form a vertical stack of medical devices 20 which is fixed for example to a stand 4. The stand 4 may comprise wheels such that the stand 4 at least to some extend is movable with respect to the patient's bed B or together with the patient's bed B. The stand 4 may comprise a pole 40 to which the rack 2 for carrying the medical devices 20 is attached and comprises, at its top end, fastening means in the shape of hooks to fasten a number of containers 6 containing medication or nutrients or other fluids to be administered to the patient.

The rack 2 serves to arrange the medical devices 20 in an organized fashion at the bedside of the patient. The rack 2 herein provides a power supply for the medical devices 20, ensures a secure and reliable fixation of the medical devices 20, and provides a communication of the medical devices 2 among each other and with an external communication network and with external periphery devices such as a nurse call, a printer, a computer, a monitor or the like.

Conventionally, the medical devices 20 can be fixed to the rack 2 and for this are mechanically and electrically connected to the rack 2 such that via the rack 2 each medical device 20 can be supplied with power and may communicate with other medical devices 20 and with external devices and/or an external communication network. The rack 2 hence serves as a communication spine providing a communication facility and an electric power supply and embedding the medical devices 20 into a hospital environment including a hospital communication network and a hospital management system.

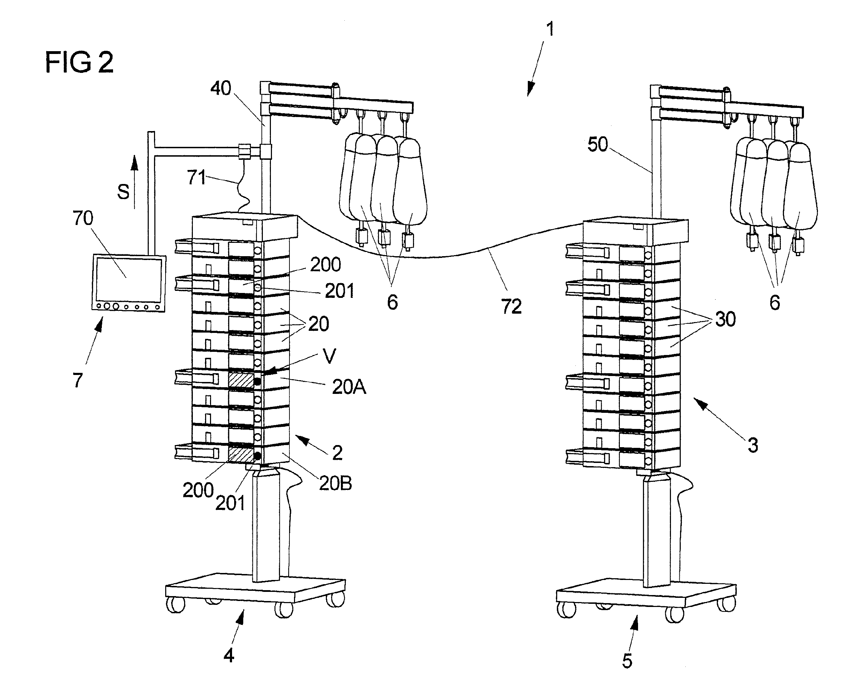

Fig. 2 shows a system 1 comprising multiple organization devices 2, 3 in the shape of racks, each rack being constituted to receive a multiplicity of medical devices 20, 30 to arrange the medical devices 20, 30 as vertical stacks. The organization devices 2, 3 are arranged on poles 40, 50 of individual stands 4, 5 and may together be placed at the bed side of a patient in a hospital environment.

The medical devices 20, 30 may, in particular, be constituted as infusion devices such as syringe pumps or volumetric pumps. The medical devices 20, 30 hence serve to administer medical fluids such as drugs, nutritional fluids or any other fluid to a patient. For this, infusion lines 21 (see Fig. 1) may extend from the medical devices 20, 30 towards a patient and towards infusion bags 6 such that medical fluids may be delivered from the infusion bags 6 to the patient.

The medical devices 20, 30 are controlled by means of a central control device 7, which in the shown embodiment is arranged on one of the stands 4 and 5 and via communication lines 71, 72 is communicatively connected to the organization devices 2, 3 and the medical devices 20, 30 arranged thereon. The control device 7 may exchange data with the organization devices 2, 3 and the medical devices 20, 30 arranged thereon, such that control commands may be transmitted from the control device 7 towards the medical devices 20, 30 and operational data may be transmitted from the medical devices 20, 30 towards the control device 7 for example for displaying on a display 70 of the control device 7.

The display 70 of the control device 7 may, for example, be a touch-sensitive display and may also serve as an input device allowing a user to enter control commands into the control device 7. Via the control commands operational settings of the medical devices 20, 30 arranged on the organization devices 2, 3 may be adapted, an infusion process may be started or ended or other settings of the medical devices 20, 30 may be configured.

Each of the medical devices 20, 30 comprises a display 200 and one or multiple control buttons or knobs 201, for example rotary knobs or the like. Via the display 200, for example operational information may be displayed directly at the medical devices 20, 30, wherein the display 200 may be constituted as a touch-sensitive display such that via the display 200 control commands relating to a medical device 20, 30 may be entered directly at the medical device 20, 30. Via the one or the multiple control buttons or knobs 201 it likewise is possible to enter commands at the medical devices 20, 30 or to change operational settings directly at the medical devices 20, 30.

In a typical scenario a user will primarily enter control commands via the central control device 7 and its touch-sensitive display 70. The display 70 herein allows a user to select one or multiple medical devices 20, 30 for entering control commands in relation to the selected medical devices 20, 30.

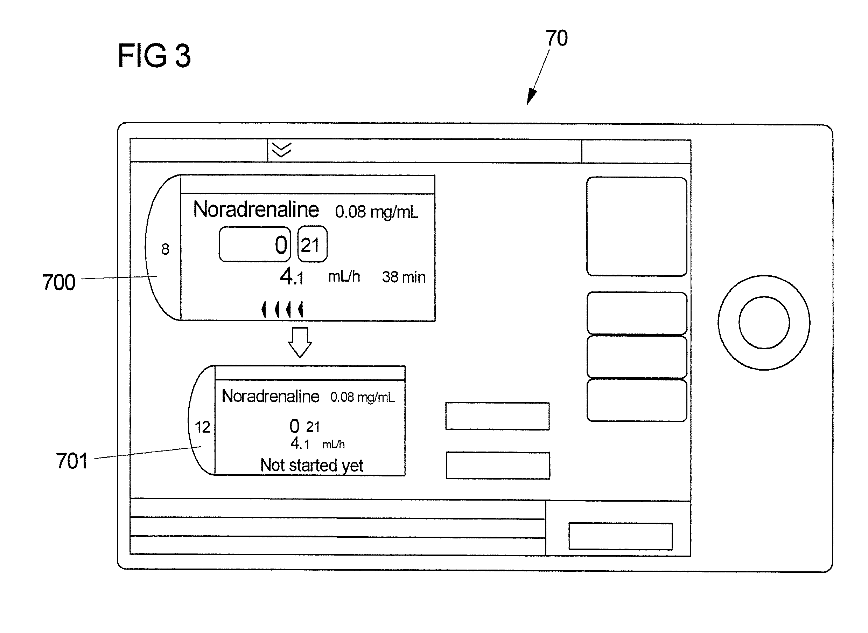

This is shown in an example in Fig. 3. In the example of Fig. 3, a user has selected medical devices 20, 30 having the indices "8" and "12" for performing an infusion process, wherein the infusion process starts with the medical device having the index "8", followed by the medical device having the index "12".

The medical device having the index "8" in the screen view of Fig. 3 relates to the physical medical device 20A at the eighth position (counted from the top) on the organization device 4 shown at the left in Fig. 2. The medical device having the index "12" in turn relates to the physical medical device 20B at the twelfth position (counted from the top) at the left organization device 4 shown in Fig. 2.

In particular in scenarios in which multiple organization devices 4, 5 each carrying a multiplicity of medical devices 20, 30 are used in combination a complex system 1 arises which may make it hard for a user to associate a selection on the display 70 of the control device 7 with the actual physical medical devices 20A, 20B physically arranged on the organization devices 4, 5.

Hence, to facilitate the association of a selection on the display 70 of the control device 7 with physical medical devices 20A, 20B arranged on the organization devices 4, 5, upon selection of an individual medical device 20A, 20B or a group of medical devices 20A, 20B on the display 70 of the control device 7 the control device 7 generates a control signal S and transmits it to the organization devices 2, 3 and the medical devices 20, 30 arranged thereon. The control signal S causes for example those medical devices 20A, 20B which have been selected by the user to output a visual signal such that the user easily and intuitively can make the association between the physical medical devices 20A, 20B which have been selected and the selection made on the display 70 of the control device 7.

In the particular example of Fig. 2 and 3, medical devices 20A, 20B have been selected. Hence, both medical devices 20A, 20B show visual signals V differentiating them from the other medical devices 20, 30 such that a user may easily make out the physical medical devices 20A, 20B which were selected.

The visual signal V displayed on each selected medical device 20A, 20B may be chosen from a multiplicity of possible visual signals.

For example, a brightness value of the display 200 of each selected medical device 20A, 20B may be adjusted such that the displays 200 of the selected medical devices 20A, 20B appear brighter than the displays 200 of the non-selected medical devices 20, 30. For this, the brightness value of the displays 200 of the selected medical devices 20A, 20B may be increased beyond a default brightness value, whereas in contrast the brightness of the displays 200 of the non-selected medical devices 20, 30 is decreased beneath the default brightness value.

In a default state the displays 200 of the medical devices 20, 30 on the organization devices 2, 3 may be controlled by the control device 7 to have a homogeneous default brightness. By increasing the brightness of the selected medical devices 20A, 20B and/or by dimming the brightness of the displays 200 of the non-selected medical devices 20, 30, hence, a visual effect at the medical devices 20, 30 may be caused clearly distinguishing the selected medical devices 20A, 20B from the non-selected medical devices 20, 30.

In addition or alternatively, the color of the displays 200 of the selected medical devices 20A, 20B may be changed, or the contrast may be altered.

Further in addition or alternatively, a light signal may appear at the button or knob 201 of the selected medical devices 20A, 20B such that the button or knob 201 shines up. The light signal at the button or knob 201 may be a white-light signal or a color-light signal.

The visual signal V may be constant at least over a predetermined period of time. Alternatively, the visual signal V may be time-varying. For example, the visual signal V may periodically change such that a blinking impression at the display 200 or the button or knob 201 arises.

For example, a rectangular box surrounding the display 200 or at least a portion of the display 200 may appear and may blink periodically to indicate the selected medical devices 20A, 20B.

The visual signal V may for example appear for a predetermined amount of time and may, after lapse of the predetermined amount of time, automatically disappear. Alternatively, the visual signal V may disappear after a user enters a cancellation command at the control device 7.

The idea underlying the invention is not limited to the embodiments described above.

In particular, the organization devices may have a different shape and constitution than described above. Generally, the organization devices serve to arrange a multiplicity of medical devices in an organized fashion, but not necessarily form a vertical stack of medical devices.

The control device may be a separate unit from the organization devices, or may also be integrated into one of the organization devices or into one of the medical devices.

The medical devices may have the shape of infusion devices such as syringe pumps or volumetric pumps. However, also other medical devices other than infusion devices may be used.

### List of Reference Numerals

- 1: System
- 2, 3: Organization device
- 20, 20A, 20B, 30: Medical device
- 200: Display
- 201: Button
- 21: Infusion lines
- 4, 5: Stand
- 40, 50: Pole
- 6: Infusion bags
- 7: Control device
- 70: Input device (touch-sensitive display)
- 71, 72: Communication line
- B: Patient's bed
- S: Control signal
- V: Visual signal

## Claims

1. System (1), comprising
- at least one organization device (2, 3) for arranging a multiplicity of medical devices (20, 30) and for providing a communication link for the multiplicity of medical devices (20, 30),
- a multiplicity of medical devices (20, 30) arranged on the at least one organization device (2, 3), each medical device (20, 30) comprising a display (200) for displaying operational information of the medical device (20, 30), and
- a control device (7) being communicatively connected with the at least one organization device (2, 3) and the multiplicity of medical devices (20, 30) arranged on the at least one organization device (2, 3), wherein the control device (7) comprises an input device (70) for allowing to select by a user at least one of the multiplicity of medical devices (20, 30) for inputting a control command for controlling the selected at least one medical device (20A, 20B),
**characterized in**
**that** the control device (7) is constituted to generate and transmit, upon selection of at least one of the multiplicity of medical devices (20, 30) via the input device (70) of the control device (7), at least one control signal (S) for causing a visual signal (V) to appear at the selected at least one medical device (20A, 20B) and/or at the other, non-selected medical devices of the multiplicity of medical devices (20, 30) to visually differentiate the selected at least one medical device (20A, 20B) from the other, non-selected medical devices.

2. System (1) according to claim 1, **characterized in that** the control device (7) is constituted to set a brightness value of the display of each of the multiplicity of medical devices (20, 30) to a default brightness value.

3. System (1) according to claim 2, **characterized in that** the default brightness value is user selectable via the control device (7).

4. System (1) according to claim 2 or 3, **characterized in that** the brightness value relates to a backlight brightness of the display of each of the multiplicity of medical devices (20, 30).

5. System (1) according to one of claims 2 to 4, **characterized in that** the at least one control signal (S) causes the brightness value of the selected at least one medical device (20A, 20B) to be increased beyond the default brightness value.

6. System (1) according to one of claims 2 to 5, **characterized in that** the at least one control signal (S) causes the brightness value of the other, non-selected medical devices to be decreased beneath the default brightness value.

7. System (1) according to one of the preceding claims, **characterized in that** the visual signal (V) includes the displaying of a visual mark on the display (200) of the selected at least one medical device (20A, 20B).

8. System (1) according to claim 7, **characterized in that** the visual signal (V) includes a rectangular box surrounding at least a portion of the display (200).

9. System (1) according to one of the preceding claims, **characterized in that** the visual signal (V) includes the lighting of at least one button of the selected at least one medical device (20A, 20B).

10. System (1) according to one of the preceding claims, **characterized in that** the visual signal (V) is time-varying, in particular flashing.

11. System (1) according to one of the preceding claims, **characterized in that** the visual signal (V) disappears after a predetermined time period or after inputting, by a user, a cancellation command at the control device (70).

12. Method for operating a system (1), the system (1) comprising
- at least one organization device (2, 3) for mechanically holding a multiplicity of medical devices (20, 30) and for providing a communication link for the multiplicity of medical devices (20, 30),
- a multiplicity of medical devices (20, 30) arranged on the at least one organization device (2, 3), each medical device (20, 30) comprising a display (200) for displaying operational information of the medical device (20, 30), and
- a control device (7) being communicatively connected with the at least one organization device (2, 3) and the multiplicity of medical devices (20, 30) arranged on the at least one organization device (2, 3),
wherein a user selects, via an input device (70) of the control device (7), at least one of the multiplicity of medical devices (20, 30) for inputting a control command for controlling the selected at least one medical device (20, 30),
**characterized in**
**that** the control device (7) generates and transmits, upon selection of at least one of the multiplicity of medical devices (20, 30) via the input device (70) of the control device (7), at least one control signal (S), wherein the control signal (S) causes a visual signal (V) to appear at the selected at least one medical device (20A, 20B) and/or at the other, non-selected medical devices of the multiplicity of medical devices (20, 30) to visually differentiate the selected at least one medical device (20A, 20B) from the other, non-selected medical devices.
